Europäisches Patentamt

**European Patent Office**

Office européen des brevets

(19)

(11) Numéro de publication: **0 151 560**
**A1**

(12) **DEMANDE DE BREVET EUROPEEN**

(21) Numéro de dépôt: **85870001.6**

(22) Date de dépôt: **02.01.85**

(51) Int. Cl.⁴: **C 12 N 15/00**

(30) Priorité: **12.01.84 BE 212201**

(43) Date de publication de la demande:
**14.08.85 Bulletin 85/33**

(84) Etats contractants désignés:
**AT BE CH DE FR GB IT LI LU NL SE**

(71) Demandeur: **Région Wallonne représentée par l'Exécutif Régional Wallon**
**11, Boulevard de l'Empereur**
**B-1000 Bruxelles(BE)**

(72) Inventeur: **Jacobs, Paul**
**Chemin des Crolithes, 119**
**B-7806 Lanquesaint(BE)**

(72) Inventeur: **Bollen, Alex Joseph**
**Gaasbeekstraat 65**
**B-1711 Itterbeek(BE)**

(72) Inventeur: **Van Elsen, Ary**
**Rue du Midi 147**
**B-1000 Bruxelles(BE)**

(72) Inventeur: **Herzog, Albert Alfred Maurice**
**Elewijtsesteenweg 190**
**B-1840 Eppegem(BE)**

(72) Inventeur: **Morais Cravador, Alfredo Jaime**
**Avenue des Cygnes, 21**
**B-1640 Rode-St.-Genese(BE)**

(74) Mandataire: **Tasset, Gérard**
**SMITHKLINE - RIT rue de l'Institut, 89**
**B-1330 Rixensart(BE)**

(54) **Procédé de préparation de clones bactériens portant une information génétique optimalisée pour la protection du facteur de déclenchement de l'hormone de croissance humaine dans Escherichia coli.**

(57) Procédé de préparation de clones bactériens portant une information génétique optimalisée pour la production de GRF humain dans *E. coli*, caractérisé en ce qu'on sélectionne parmi l'ensemble des séquences d'ADN potentiellement codantes pour le GRF une séquence considérée comme optimale pour l'expression du GRF dans *E. coli*, on découpe cette séquence en fragments simple brin de façon optimale compte tenu des possibilités de synthèse chimique d'ADN et des exigences de spécificité des hybrides à former entre ces fragments d'ADN, on synthétise chimiquement les fragments dont l'ensemble couvre l'ADN-GRF, on hybride et on ligue *in vitro* ces fragments entre eux pour obtenir l'ADN-GRF, on insère l'ADN-GRF obtenu dans un vecteur plasmidique, on transforme des bactéries hôtes *Escherichia coli* par l'ADN hybride obtenu dans la phase précédente, on sélectionne parmi ces bactéries transformées celles qui portent un plasmide de manière à obtenir une banque de clones, on isole de cette banque les clones porteurs de l'ADN-GRF par crible caractéristique de l'ADN-GRF, et on caractérise l'ADN-GRF par séquençage.

EP 0 151 560 A1

## PROCEDE DE PREPARATION DE CLONES BACTERIENS PORTANT UNE INFORMATION GENETIQUE OPTIMALISEE POUR LA PRODUCTION DU FACTEUR DE DECLENCHEMENT DE L'HORMONE DE CROISSANCE HUMAINE DANS ESCHERICHIA COLI

La présente invention se rapporte à la préparation de clones bactériens portant une information génétique codant pour la production du facteur de déclenchement de l'hormone de croissance humaine, choisie en vue d'optimaliser son expression dans E. coli et de s'adapter à des vecteurs d'expression particuliers. Elle comprend plus particulièrement la conception d'une séquence nucléotidique pour s'exprimer de façon optimale dans E. coli; la synthèse chimique de fragments d'ADN monobrin représentant l'ensemble des deux brins de la séquence précitée; l'hybridation et la ligation entre eux et avec le plasmide vecteur de ces fragments : le clonage de l'ensemble dans E. coli et la sélection et la caractérisation de clones portant l'information dont question.

Le facteur de déclenchement de l'hormone de croissance humaine, également appelé somatocrinine, désigné ci-après par l'abréviation GRF, est un régulateur positif de la sécrétion de l'hormone de croissance, désignée ci-après par l'abréviation GH, par l'adénohypophyse. Il s'agit d'un polypeptide de 44 acides aminés, isolé et séquencé à partir d'une tumeur pancréatique humaine causant l'acromégalie. Des anticorps dressés contre ce peptide ont permis de mettre en évidence un matériel immuno-réactif dans l'hypothalamus de différents primates; de plus, un polypeptide apparemment identique a été isolé de l'hypothalamus humain. Enfin, des expériences de type "southern blot" indiquent qu'il n'y aurait qu'un seul gène codant pour le GRF. Ceci suggère donc que le facteur pancréatique tumoral est bien codé par le même mRNA que le facteur physiologique hypothalamique. L'utilité de disposer de quantités abondantes de GRF humain résulte notamment de ce que sa déficience d'origine génétique ou physiologique est cause de nanisme; de ce que son effet stimulateur sur la synthèse de GH doit conduire à l'utiliser pour le diagnostic des déficiences ou dérèglements du métabolisme de la GH; de ce que son administration pourrait permettre une accélération de la régénération de tissus par exemple dans le traitement de grands brûlés et de ce qu'il a été montré que l'administration de GRF humain à des animaux stimule leur croissance.

La présente invention a pour objet la préparation de clones bactériens portant l'information génétique considérée comme optimale pour la production de GRF dans E. coli à partir de fragments de DNA synthétiques hybrides, ligués et clonés dans une bactérie hôte.

Ce procédé de préparation d'un clone bactérien portant l'information optimalisée pour la production de GRF dans E. coli selon la présente invention est caractérisé en ce que :

a) on sélectionne parmi l'ensemble des séquences d'ADN potentiellement codantes pour le GRF une séquence considérée comme optimale pour l'expression du GRF dans E. coli;

b) on découpe cette séquence en fragments simple brin de façon optimale compte tenu des possibilités de synthèse chimique d'ADN et des exigences de spécificité des hybrides à former entre ces fragments d'ADN;

c) on synthétise chimiquement les fragments dont l'ensemble couvre l'ADN-GRF;

d) on hybride et on ligue in vitro ces fragments entre eux pour obtenir l'ADN-GRF;

e)   on insère l'ADN-GRF obtenu dans un vecteur plasmidique;

f)   on transforme des bactéries hôtes Escherichia coli par l'ADN hybride obtenu dans la phase précédente;

g)   on sélectionne parmi ces bactéries transformées celles qui portent un plasmide de manière à obtenir une banque de clones;

h)   on isole de cette banque les clones porteurs de l'ADN-GRF par crible caractéristique de l'ADN-GRF;

i)   on caractérise l'ADN-GRF par séquençage.

Le procédé faisant l'objet de la présente invention est plus particulièrement caractérisé par le choix des moyens suivants :

a)   on sélectionne, parmi l'ensemble des séquences d'ADN potentiellement codantes pour le GRF, celle où à chaque acide aminé correspond le codon le plus utilisé chez E. coli;

b)   on découpe cette séquence en fragments simple brin de façon à ce que les hybrides voulus entre deux fragments couvrent au moins 10 bases et à ce que les hybrides non désirables ne couvrent pas plus de 6 bases;

c)   on synthétise chimiquement des fragments de taille comprise entre 26 et 28 bases sauf pour les fragments des extrémités de l'ADN-GRF;

d)   on hybride et on ligue in vitro ces fragments entre eux après avoir phosphorylé en 5' les fragments qui ne seront pas directement ligués au vecteur plasmidique;

e)   on insère l'ADGRF obtenu dans un dérivé de PBR322;

f)   on transforme des bactéries de la souche E. coli ATCC 31446 par l'ADN hybride obtenu dans la phase précédente;

g)   on sélectionne par leur résistance à un antibiotique les bactéries qui portent un plasmide;

h) on isole de cette banque les clones porteurs de l'ADN-GRF par hybridation avec des fragments constitutifs de l'ADN-GRF marqués au $^{32}$P;

i) on caractérise l'ADN-GRF par séquençage au moyen de la méthode de Maxam et Gilbert à partir d'un site de restriction proche de l'ADN-GRF dans le plasmide.

Le procédé de préparation d'un clone bactérien portant une information génétique optimalisée pour la production du facteur de déclenchement de l'hormone de croissance humaine dans l'Escherichia coli est caractérisé par la combinaison et le choix d'un ensemble de moyens détaillés ci-après.

a) La définition de la séquence d'ADN optimale pour la production de GRF humain par E. coli peut être faite par exemple, en choisissant pour chaque acide aminé à encoder le triplet le plus généralement utilisé dans la bactérie. On trouvera dans "Ikemura, T., Nucleic Acid Research 11, 143-144 (1982)" et "Gouy, P. and Goutier, C., Nucleic Acid Research 10, 7055-7074 (1982)" l'état des connaissances quant à l'usage des codons chez E. coli. Il pourra également être tenu compte de possibilités d'hybridations non souhaitables entre fragments en changeant certaines des bases impliquées sans que ce changement entraîne une modification des acides aminés codés.

b) La séquence d'ADN double brin définie par la méthode décrite en a) ne peut être obtenue que par synthèse chimique de fragments d'ADN simple brin. Le découpage de la séquence complète en ses fragments constitutifs doit répondre notamment aux critères suivants : il doit être tel que les segments monobrins soient de taille compatible avec les possibilités actuelles de la synthèse chimique d'ADN (c'est-à-dire pas plus de 30 bases environ), possibilités que l'on peut trouver décrites dans "Narang, S.A., Tetrahedron 39, 3-22 1983".

De préférence, on choisit les fragments tels que seules les hybridations escomptées puissent se réaliser. Ceci peut être testé a priori par essai à l'ordinateur de toutes les hybridations possibles entre fragments et sous-fragments. De nombreux programmes d'ordinateur existent à cette fin. L'état de l'art en cette matière peut être trouvé dans "The applications of computers to research on Nucleic Acids" (Söll, D. and Roberts, R.J. eds., IRL Press, Oxford and Washington D.C., 1982).

c) La synthèse chimique d'ADN peut se faire selon différentes méthodes dont on trouvera la description dans "Narang, S.A. Tetrahedron 39 (1983) 3-22; Itakura, K., TIBS (1982) 442-445; Ohtruka, E., Ikehara, M. and Söll, D., N.A.R. 10 (1982) 6553-6570".

A titre d'exemple, on peut utiliser soit la méthode des phosphotriesters décrite par Brown, E.L., Belagaje, R., Ryan, M.J. and Khorana, H.G. (Methods in Enzymology 68 (1979) 109), soit celle des phosphite triesters décrite par Letsinger, R.L. and Lunsford, W.B. (J. Am. Chem. Soc. 98 (1976) 3655).

Ces méthodes diffèrent par le degré d'oxydation de l'atome de phosphore dans les intermédiaires nucléotidiques mis en oeuvre lors de la réaction de couplage.

La synthèse peut se faire soit en phase liquide, soit sur support solide; dans ce cas, différents supports sont possibles, par exemple de la silice, des billes de polystyrène ou des billes de verre. Leur utilisation est décrite dans Izo, H., Ike, Y., Ikuta, S. and Itakura K. (Nucleic Acid Research 10, (1982) 1755); Efimov, V.A., Reverdato, S.V. and Chakhmakhcheva, O.G., Nucleic Acid Research 10 6675 (1982); Hattar, J.B., Rayner, B. and Imbach, J.L., Nucleosides et Nucleotides 1, 289 (1982).

d) L'hybridation et la ligation des fragments d'ADN synthétique entre eux peut se faire par différentes méthodes dont on trouvera la description dans Molecular Cloning (1982 - Maniatis, T., Fritsch, E.F. and Sambrook, J., CSH Publ.). Ces méthodes sont basées sur le principe de l'hybridation des séquences complémentaires d'ADN. Elles consistent à mélanger les fragments d'ADN dans des conditions de température et de force ionique adéquate à la formation des hybrides spécifiques. La ligation de ces fragments hybridés est le résultat de l'action d'une enzyme, par exemple la T4 DNA ligase.

e) La formation in vitro de molécules hybrides entre l'ADN complémentaire double brin à cloner et l'ADN de vecteurs plasmides s'effectue selon le procédé décrit dans Molecular Cloning (1982 - Maniatis, T., Fritsch, E.F. and Sambrook, J., CSH Publ.), qui consiste à mélanger les molécules dans des conditions de température et de force ionique appropriées. C'est ce protocole qui a été suivi dans la réalisation de l'invention. L'ADN vecteur employé dans la réaction de recircularisation in vitro peut provenir de différents plasmides. Parmi les plasmides les plus couramment utilisés, on rencontre le plasmide pBR322 qui contient deux sites d'insertion possible, PstI et BamHI. Ces deux sites conviennent à l'insertion de l'ADN susmentionné (Bolivar, F., Rodriguez, R.L., Greene, P.J., Betlach, M.C., Heynecker, H.L., Boyer, H.W., Crosa, J.H. and Falkow, S., Gene 2 (1977) 95). Les dérivés du plasmide pBR322 peuvent aussi convenir. Dans la réalisation de cette invention, on choisit par exemple le plasmide PCQV2 (Queen, C., J. of Mol. and Applied Genet. 2, 1-10, 1983) dans lequel on clone au site BamHI. Les extrémités cohésives d'ADN simple brin ainsi générées sont alors digérées au moyen d'une enzyme, par exemple la Mungbean nucléase.

f) La plupart des méthodes utilisées pour transformer des bactéries par de l'ADN exogène font appel à un traitement particulier des bactéries par du chlorure de calcium et visent à optimiser l'efficacité de transformation pour différentes souches de bactéries. Dans le cadre de l'invention, plusieurs types de bactéries peuvent servir d'hôtes aux vecteurs utilisés. On prendra de préférence une souche d'_Escherichia_ dont les conditions de croissance sont particulièrement commodes, par exemple la souche _E. coli_ K12 MM294 (ATCC 31446), _E. coli_ B et _E. coli_ X1776 (ATCC 31537).

En outre, parmi les différents procédés de transformation, on choisit celui décrit dans Mandel, M. and Higa, A. (1970, J. Mol. Biol. _53_, 154).

g) En raison des marqueurs de résistance aux antibiotiques présents dans l'ADN du vecteur, il est simple d'isoler les bactéries transformées par l'ADN recombiné _in vitro_. Par exemple, si l'ADN du vecteur porte le gène $Tet^R$, toute bactérie portant un tel vecteur sera résistante à la tétracycline. En outre, si l'insertion de l'ADN dans le vecteur a pour effet d'inactiver un gène de résistance à un antibiotique, on repèrera les bactéries qui portent le vecteur recombinant par leur sensibilité à cet antibiotique. Par exemple, on choisit comme marqueur de sélection le caractère $Amp^R$ du plasmide PCQV2.

h) Le crible de sélection des clones porteurs de l'ADN-GRF peut se faire selon des méthodes mettant en oeuvre soit une sonde synthétique, soit des moyens immunologiques. Ces différentes approches sont détaillées dans Genetic Engineering, vol. 1, Williamson, R. Eds., (1981) Academic Press, N.Y. Dans le cadre de la présente invention, on utilise de préférence l'un ou plusieurs des fragments constitutifs de l'ADN-GRF comme sonde oligodésoxyribonucléotidique marqué au $^{32}P$ que l'on hybride avec les clones de la banque.

i) La séquence d'ADN créée dans l'un ou plusieurs des clones obtenus peut être caractérisée par des méthodes de séquençage décrites dans Maxam, A. and Gilbert, W., Methods in Enzymology 65 (1980) 497-559 et dans Sanger, F., Nicklen, S. and Coulson, A.R., Proc. Natl. Acad. Sci. USA 74 (1977) 5463. Dans le cadre de la présente invention, on choisira de préférence la méthode de Maxam et Gilbert.

L'exemple non limitatif suivant qui s'applique au clonage de l'ADN-GRF dans le plasmide PCQV2 permettra de mieux comprendre l'invention.

1. **Choix de la séquence optimale pour l'expression du GRF dans E. coli**

Les séquences potentiellement codantes pour le GRF sont extrêmement nombreuses du fait de la dégénérescence du code génétique. Dans le cadre de la présente invention, nous avons choisi une séquence telle que les 44 acides aminés du GRF soient encodés chacun par le codon le plus fréquemment utilisé chez Escherichia coli pour cet acide aminé.

La séquence inventée est la suivante

```
5' TACGCTGACGCTATCTTCACTAACTCTTACCGTAAAGTTCTGGGTCAGCTG
3' ATGCGACTGCGATAGAAGTGATTGAGAATGGCATTTCAAGACCCAGTCGAC
5' TCTGCTCGTAAACTGCTGCAGGACATCATGTCTCGTCAGCAGGGTGAATCT
3' AGACGAGCATTTGACGACGTCCTGTAGTACAGAGCAGTCGTCCCACTTAGA
5' AACCAGGAACGTGGTGCTCGTGCTCGTCTGTAAG 3'
3' TTGGTCCTTGCACCACGAGCACGAGCAGACATTCAGCT 5'
```

Elle est de plus caractérisée en ce que son extrémité 5' (sur la fibre identique au mRNA) est "blunt" et correspond à la première base du deuxième codon du GRF et en ce que son extrémité 3' (sur la fibre codante) se termine, en phase après le triplet codant pour le dernier acide aminé du GRF par un triple "stop" (TAA), lui-même suivi de la partie d'un site de restriction SalI telle qu'elle apparaît après clivage d'un ADN par cette enzyme.

2. Découpage de cette séquence en fragments d'ADN monobrin

La subdivision de cette séquence en des fragments monobrins convenant pour la synthèse chimique et adaptés aux exigences d'une reconstitution non ambiguë de l'ADN-GRF par hybridation et ligation, peut être faite de différentes façons. Dans le cadre de la préseente invention, on choisira de préférence le découpage en les fragments suivants :

A27 : 5' TACGCTGACGCTATCTTCACTAACTCT

B27 : 5' TACCGTAAAGTTCTGGGTCAGCTGTCT

C27 : 5' GCTCGTAAACTGCTGCAGGACATCATG

D27 : 5' TCTCGTCAGCAGGGTGAATCTAACCAG

E28 : 5' GAACGTGGTGCTCGTGCTCGTCTGTAAG

F14 : 5' ATAGCGTCAGCGTA

G27 : 5' AGAACTTTACGGTAAGAGTTAGTGAAG

H27 : 5' AGCAGTTTACGAGCAGACAGCTGACCC

I26 : 5' CCTGCTGACGAGACATGATGTCCTGC

J27 : 5' GAGCACCACGTTCCTGGTTAGATTCAC

K19 : 5' TCGACTTACAGACGAGCAC

3. Synthèse des fragments d'ADN monobrin constitutifs de l'ADN-GRF

La synthèse du fragment F14 a été réalisée sur support solide de polystyrène en utilisant un monomère et six dimères protégés préparés en solution par une combinaison de différents procédés décrits ou modifiés. Les autres fragments ont été synthétisés sur un support mixte de polydiméthylacrylamide-silice dans une colonne sous flux continu selon la méthode décrite par Gait, M.J., Matthes, H.W.D., Singh, M. et Titmas, R.C. (J. Chem. Soc. Chem. Comm. p 37, 1982), en utilisant uniquement des dimères ou un monomère et des dimères selon que le fragment contient un nombre impair ou pair de désoxyribonucléotides.

Les désoxyribonucléotides portant le groupe protecteur diméthoxytrityle ont été purifés d'abord sur Sephadex G-50 en éluant avec du TEAB $10^{-2}$M pH 7,0 et ensuite deux fois par HPLC sur colonne Radial Pak RPH C18 10 μm avant et après déprotection, en utilisant le TEAA 50 mM pH 7 plus acétonitrile comme éluant et des gradients de 15 % à 30 % d'acétonitrile en 20' et de 0 % à 20 % d'acétonitrile en 20' respectivement, et un débit de 2 ml/min. Les rendements finaux obtenus sont de l'ordre de 8 %.

4. Hybridation et ligation

La ligation par la T4 DNA ligase des fragments d'ADN hybridés exige que ceux-ci soient phosphorylés sur leur extrémité 5'. Dans le cadre de la présente invention, celle-ci se fera de préférence par la T4-polynucléotide kinase. Différentes méthodes sont décritess dans "Molecular Cloning" (Maniatis, T., Fritsch, E.F., Sambrook, J., CSH, Ed. 1982).

De préférence, on utilise le protocole suivant : à 100 micomoles de chaque désoxyoligonucléotide lyophilisé sont ajoutés 2 μl d'ATP 0,1 mM; 2 μl de $^{32}$P ATP 3,3 mM à 3000 Ci par mM; 3 μl de tampon Tris HCl 660 mM pH 7,8, $MgCl_2$ 66 mM, ß mercaptoéthanol 160 mM; 5 unités de T4 polynucléotide kinase, soit 0,5 μl et 22,5 μl d'eau. Le mélange réactionnel est incubé 1 h à 37° C puis fractionné sur une colonne de 5 ml de Sephadex G50. Les fractions contenant l'ADN marqué sont rassemblées, lyophilisées et resuspendues dans $H_2O$. De préférence les fragments A et K ne sont pas phosphorylés pour éviter la formation de polymères d'ADN-GRF.

50 pmoles de chaque fragment sont mélangées, lyophilisées et resuspendues dans 49,5 μl de tampon TrisHCl 50 mM 7,4, $MgCl_2$ 10 mM, DTT 10 mM, spermidine 1 mM.

Le mélange est chauffé à 90° C pendant 2' puis refroidi lentement jusque 4° C. Sont alors ajoutés : 1 μl d'ATP 50 mM, 0,5 μl de Serum Albumine Bovine (BSA) à 10 mg/ml et 10 U soit 10 μl de T4 DNA ligase.

Le mélange est incubé 25 h à 16° C. Le produit peut être purifié, de préférence par fractionne- ment sur gel d'acrylamide 7,5 % dont on élue une zone correspondant à un ADN de taille attendue, soit en l'occurrence 140 paires de bases environ.

5. <u>Fabrication in vitro de recombinants entre l'ADN-GRF et l'ADN du vecteur PCQV2.</u>

L'insertion de l'ADN-GRF dans le plasmide PCQV2 impose de préparer le vecteur de façon à ce qu'il présente un signal de départ de la traduction (ATG) dont le G est la dernière base d'une extrémité "Blunt" d'une part et dont l'autre extrémité soit la fraction restante d'un site de restriction <u>SalI</u> produite par clivage du vecteur par cette enzyme.

L'état de l'art pour la réalisation de cette étape peut se trouver dans "Molecular Cloning" (Maniatis, T., Fritsch, E.F., Sambrook, J., CSH Ed. 1982).

On mélange 100 ng (soit 0,033 picomoles) du vecteur préparé comme décrit avec l'ADN-GRF, les deux sont ligués comme décrit en 4.

6. <u>Clonage dans la souche d'Escherichia coli MM294</u>

On procède alors à la transformation de la souche MM294 ("Molecular Cloning", Maniatis, T., Fritsch, E.F., Sambrook, J., CSH Ed. 1982) dont le système de restriction est modifié de façon à tolérer la présence d'un ADN étranger selon la méthode décrite dans "Molecular Cloning" (Maniatis, T., Fritsch, E.F. and Sambrook, J., CSH Ed. 1982).

7. Obtention de la banque de clones

En raison du caractère Amp$^R$ du plasmide, on peut sélectionner les bactéries transformées par simple croissance sur milieu contenant de l'ampicilline.

L'ensemble des manipulations décrites ci-dessus a conduit à l'obtention d'une banque de 1500 clones dont une grande partie doit contenir des séquences d'ADN codant pour le GRF.

8. Isolement des clones porteurs de l'ADN-GRF

On a recherché les clones porteurs de l'ADN-GRF au moyen des fragments B et J utilisés comme sonde. Après avoir marqué ces sondes au $^{32}$P par une réaction enzymatique (kination en 5' de la sonde), nous les avons utilisées pour cribler la banque de clones. Pratiquement, on fixe l'ADN de chaque clone sur feuille de nitrocellulose, suivant la technique de Grunstein, M. et Hogness, D. (Proc. Natl. Acad. Sci. USA 72 (1975) 3961) et on l'hybride avec la sonde synthétique marquée au $^{32}$P. Dans des conditions ioniques et de température appropriées (0,9 M NaCl et 50° C), la sonde radio-active va reconnaître spécifiquement l'ADN des clones portant une séquence homologue, c'est-à-dire une séquence de nucléotides typique de l'ADN-GRF. On visualise les clones positifs par autoradiographie. Cette méthode a permis d'identifier 79 clones (sur 1500 analysés) portant tout ou partie de l'ADN-GRF.

9. Caractérisation de l'ADN-GRF du clone pULB1323

On caractérise l'ADN-GRF du clone pULB1323 par l'établissement de sa séquence nucléotidique. A cet effet, l'ADN du plasmide recombinant est préparé et clivé au site de restriction SalI unique dans le plasmide recombinant. Les extrémités du plasmide linéarisé sont alors marquées au $^{32}$P. On coupe ensuite le plasmide par l'enzyme EcoRI générant deux fragments dont l'un porte à son extrémité marquée l'ADN-GRF. Le fragment marqué est alors soumis à une série de réactions chimiques conduisant à la production d'oligonucléotides marqués, de tailles différentes, dont l'analyse sur gel de polyacrylamide conduit à l'établissement de la séquence en bases suivant la technique de Maxam, A.M. et Gilbert, W. (Proc. Natl. Acad. Sci. USA 74 (1977) 560). On voit que l'ADN-GRF est parfaitement conforme à la séquence définie dn 1.

Bien que la présente invention ait été décrite en détail, il est évident pour l'homme de l'art que des modifications peuvent être apportées à cette description sans sortir du cadre de l'invention.

- 14 -    0151560

REVENDICATIONS

1. Procédé de préparation de clones bactériens portant une information génétique optimalisée pour la production de GRF humain dans E. coli, caractérisé en ce qu'on sélectionne parmi l'ensemble des séquences d'ADN potentiellement codantes pour le GRF une séquence considérée comme optimale pour l'expression du GRF dans E. coli, on découpe cette séquence en fragments simple brin de façon optimale compte tenu des possibilités de synthèse chimique d'ADN et des exigences de spécificité des hybrides à former entre ces fragments d'ADN, on synthétise chimiquement les fragments dont l'ensemble couvre l'ADN-GRF, on hybride et on ligue in vitro ces fragments entre eux pour obtenir l'ADN-GRF, on insère l'ADN-GRF obtenu dans un vecteur plasmidique, on transforme des bactéries hôtes Escherichia coli par l'ADN hybride obtenu dans la phase précédente, on sélectionne parmi ces bactéries transformées celles qui portent un plasmide de manière à obtenir une banque de clones, on isole de cette banque les clones porteurs de l'ADN-GRF par crible caractéristique de l'ADN-GRF, et on caractérise l'ADN-GRF par séquençage.

2. Procédé de préparation de clones bactériens portant l'ADN-GRF suivant la revendication 1, caractérisé en ce qu'on sélectionne parmi l'ensemble des séquences d'ADN potentiellement codantes pour le GRF, celle où à chaque acide aminé correspond le codon le plus utilisé chez E. coli, on découpe cette séquence en fragments simple brin de façon à ce que les hybrides voulus entre deux fragments couvrent au moins 10 bases et à ce que les hybrides non désirables ne couvrent pas plus de 6 bases, on synthétise chimiquement des fragments de taille comprise entre 26 et 28 bases sauf pour les fragments des extrémités de l'ADN-GRF, on hybride et on ligue _in vitro_ ces fragments entre eux après avoir phosphorylé en 5' les fragments qui ne seront pas directement ligués au vecteur plasmidique, on insère l'ADN-GRF obtenu dans un dérivé de PBR322, on transforme des bactéries de la souche E. coli ATCC 31446 par l'ADN hybride obtenu dans la phase précédente, on sélectionne par leur résistance à un antibiotique, les bactéries qui portent un plasmide, on isole de cette banque les clones porteurs de l'ADN-GRF par hybridation avec des fragments constitutifs de l'ADN-GRF marqués au $^{32}$P, et on caractérise l'ADN-GRF par séquençage au moyen de la méthode de Maxam et Gilbert à partir d'un site de restriction proche de l'ADN-GRF dans le plasmide.

3. Procédé de préparation de clones bactériens portant l'ADN-GRF, suivant la revendication 1 ou 2, caractérisé en ce que le vecteur plasmidique choisi est le PCQV2.

4. Procédé de préparation de clones bactériens portant l'ADN-GRF suivant l'une quelconque des revendications 1 à 3, caractérisé en ce que l'ADN-GRF est inséré dans le site BamHI du vecteur plasmidique.

5. Procédé de préparation de clones bactériens portant l'ADN-GRF suivant l'une quelconque des revendications 1 à 4, caractérisé par l'utilisation comme crible de sondes oligodésoxyribonucléotidiques synthétiques choisies parmi les fragments constitutifs de l'ADN-GRF de façon à ce qu'elles hybrident à ses extrémités.

6. Procédé de préparation de clones bactériens portant l'ADN-GRF suivant l'une quelconque des revendications 1 à 5, caractérisé en ce que la séquence de l'ADN-GRF insérée dans le plasmide correspond à la séquence considérée comme optimale pour l'expression du GRF dans E. coli.

7. Procédé de préparation de clones bactériens portant l'ADN-GRF suivant l'une quelconque des revendications 1 à 6, caractérisé en ce que la séquence sélectionnée est la séquence suivante :

5' TACGCTGACGCTATCTTCACTAACTCTTACCGTAAAGTTCTGGGTCAGCTG

3' ATGCGACTGCGATAGAAGTGATTGAGAATGGCATTTCAAGACCCAGTCGAC


5' TCTGCTCGTAAACTGCTGCAGGACATCATGTCTCGTCAGCAGGGTGAATCT

3' AGACGAGCATTTGACGACGTCCTGTAGTACAGAGCAGTCGTCCCACTTAGA


5' AACCAGGAACGTGGTGCTCGTGCTCGTCTGTAAG 3'

3' TTGGTCCTTGCACCACGAGCACGAGCAGACATTCAGCT 5'

8. Procédé de préparation de clones bactériens portant l'ADN-GRF suivant l'une quelconque des revendications 1 à 7, caractérisé en ce que les fragments simples brins sont les suivants :

A27 : 5' TACGCTGACGCTATCTTCACTAACTCT

B27 : 5' TACCGTAAAGTTCTGGGTCAGCTGTCT

C27 : 5' GCTCGTAAACTGCTGCAGGACATCATG

D27 : 5' TCTCGTCAGCAGGGTGAATCTAACCAG

E28 : 5' GAACGTGGTGCTCGTGCTCGTCTGTAAG

F14 : 5' ATAGCGTCAGCGTA

G27 : 5' AGAACTTTACGGTAAGAGTTAGTGAAG

H27 : 5' AGCAGTTTACGAGCAGACAGCTGACCC

I26 : 5' CCTGCTGACGAGACATGATGTCCTGC

J27 : 5' GAGCACCACGTTCCTGGTTAGATTCAC

K19 : 5' TCGACTTACAGACGAGCAC

Office européen
des brevets

RAPPORT DE RECHERCHE EUROPEENNE

**0151560**
Numéro de la demande

EP  85 87 0001

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| | | | C 12 N  15/00 |
| Y | PROC. NATL. ACAD. SCI, USA, vol. 80, juillet 1983, pages 4311-4314; U. GUBLER et al.: "Cloning and sequence analysis of cDNA for the precursor of human growth hormone-releasing factor, somatocrinin" * En entier * | 1 | |
| Y | NATURE, vol. 306, 3 novembre 1983, pages 86-88, Macmillan Journals Ltd.; K.E. MAYO et al.: "Expression-cloning and sequence of a cDNA encoding human growth hormone-releasing factor" * En entier * | 1 | |
| Y | BIOLOGICAL ABSTRACTS/RRM, résumé 26002461/4, Philadelphia, US; P. BARR et al.: "The chemical synthesis and expression of genes for human growth hormone releasing factor somatocrinin" & FEDERATION PROCEEDINGS (USA), vol. 42, no. 7, résumé 1160, 1983 * En entier * | 1 | **DOMAINES TECHNIQUES RECHERCHES (Int. Cl.4)**<br><br>C 12 N |

Le présent rapport de recherche a été établi pour toutes les revendications

| Lieu de la recherche | Date d'achèvement de la recherche | Examinateur |
|---|---|---|
| LA HAYE | 22-04-1985 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82